# FASCICULE DE BREVET EUROPEEN

(11) **EP 2 374 898 B1**
(45) Date de publication et mention de la délivrance du brevet: **17.04.2013**
(21) Numéro de dépôt: 09793977.1
(22) Date de dépôt: 10.07.2009
(51) Int. Cl.: G01N 27/30, G01N 27/327, C12Q 1/25

(54) **SUPPORT DE BIOCAPTEUR**
BIOSENSORTRÄGER
BIOSENSOR SUPPORT

(30) Priorité: 11.07.2008 ES 200802126
(43) Date de publication de la demande: 12.10.2011
(73) Titulaire: Biolan Microbiosensores, S.L., 48160 Derio (Bizkaia) (ES)
(72) Inventeur: ARQUERO CAVIA, Daniel, 48170 Zamudio (Bizkaia) (ES); ALBIZU LLUVIA, Asier, 48170 Zamudio (Bizkaia) (ES)
(74) Mandataire: Urizar Barandiaran, Miguel Angel
(86) Numéro de dépôt international: PCT/ES2009/000362
(87) Numéro de publication internationale: WO 2010/004069

(56) Documents cités:
- EP-A1- 0 400 918
- GB-A- 2 054 859
- GB-A- 2 054 859
- US-A- 3 770 607
- US-A- 3 770 607
- US-A- 4 224 125
- US-A- 4 440 620
- US-A- 4 820 399

## Description

La présente invention est basée sur un dispositif servant de base pour la mise en place d'une substance enzymatique ou de toute autre nature afin de créer un biocapteur pour la détection spécifique de substances dans des milieux aqueux.

On connaît déjà dans l'état de la technique des supports semblables, mais basés sur des lames massives, de préférence circulaires, en or, charbon ou platine. Ces lames sont fabriquées en métal pur, d'une épaisseur de un ou plusieurs millimètres. La partie extérieure, où les enzymes sont déposées, est polie et est au même niveau que le cylindre en plastique où elle est logée. Dans la partie intérieure de ce cylindre en plastique, dans la face interne de la lame en métal, on place normalement une résistance réalisée en charbon en poudre pressée. L'impédance typique de ce charbon en poudre est généralement inférieure à une dizaine d'ohms. Après ce charbon pressé, on place une tige, préférablement en laiton, pour que, une fois qu'elle dépassera de l'extrémité opposée à la lame en or, à travers le cylindre en plastique qui la loge, on puisse recevoir le signal électrique provenant de l'enzyme déposée sur la lame.

La lame, de préférence en or, a un diamètre compris entre 3 et 6 mm.

Ou connaît aussi dans l'état de la technique par les brevets GB2054859, US4224125 et US3770607 des supports semblables.

Par le brevet GB 2054859 est connu un Support de biocapteur, qui se compose de :
- un cylindre (3) en matériel non-conducteur d'électricité, qui dispose d'un orifice passant coaxial avec l'axe du cylindre (3)
- une tige en métal (4) disposée dans cet orifice (2) avec son extrémité supérieure placée au ras de la base supérieure du cylindre (3)
- une couche/plaque/revêtement en métal noble (4), disposée sur cette base supérieure, et
- un protecteur constitué par un film perméable de polipropilene (6) disposé sur la couche/revêtement en métal noble (4).

Ce type de supports pour la construction de biocapteurs présente l'inconvénient que le signal électrique provenant de la réaction chimique de l'enzyme est très faible. Les essais faits avec des enzymes, de la gluconase de préférence, pour la détermination de l'acide gluconique, ont démontré que, pour une lame de 3 mm de diamètre et de 0,1 mm d'épaisseur, la réponse à l'enzyme était d'à peine 5 nanoampères.

En augmentant le diamètre de la lame en or à 5 mm de diamètre, le signal a à peine augmenté de 2 nanoampères de plus.

Le biocapteur construit de cette façon, une fois introduit dans le circuit électronique potentiostatique, présente des problèmes de mesure à cause du signal de réponse électrique de l'enzyme très bas, et, en outre, une augmentation de la température du milieu aqueux où la mesure est réalisée fausse les données en variant approximativement de 2 nanoampères par chaque degré centigrade. Ceci nous oblige à maintenir rigoureusement le milieu aqueux à une température la plus constante possible.

La présente invention résout ce problème de réponse faible du signal de l'enzyme en présence de la substance chimique pour laquelle elle a été conçue. De même, elle est valable pour toute substance enzymatique, étant donné que la réponse haute au signal vient principalement de la manière dont est construit le support objet de ce brevet.

Les substances enzymatique nécessitent un métal noble ou un charbon, sur lesquels les placer pour donner une réponse à la substance pour laquelle il a été conçu. Ce support, en or de préférence, ne peut pas directement être relié par un câble ou une tige conductrice d'électricité au circuit électronique potentiostatique. Il est nécessaire de placer une impédance qui, dans les capteurs commerciaux jusqu'à présent, est effectuée avec du charbon pressé. Ce charbon doit être en contact direct avec la lame, principalement en or, et on ne peut pas placer de résistance électronique conventionnelle. Mais comme la lame en or où est déposée l'enzyme est parfaitement conductrice d'électricité, le signal électronique que produit l'enzyme est masqué par rapport au signal parasite haut ou de base produit par le circuit électronique potentiostatique.

Le support de biocapteur objet de l'invention se caractérise par le fait qu'il se compose de :
a) un cylindre en matériel non-conducteur d'électricité, qui dispose d'un orifice passant coaxial avec l'axe du cylindre
b) une tige en métal, disposée dans cet orifice avec son extrémité supérieure au ras de la base supérieure du cylindre
c) une couche/revêtement en métal noble, de entre 1 et 1000 nanomètres, disposée sur cette base supérieure
d) un protecteur en matériel non-conducteur d'électricité, disposé sur la couche/revêtement en métal noble dans la zone de jonction de cette couche avec la tige en métal et en recouvrant une surface de diamètre plus élevé que le diamètre de la tige.

La présente invention résout le problème du signal bas que donne l'enzyme déposée sur le support du biocapteur en présence de la substance chimique pour laquelle il a été conçu. En outre, il réduit énormément le signal parasite ou de ligne de base produit par le circuit électronique potentiostatique. Étant donné la topologie appliquée à sa fabrication, le prix de fabrication est environ 300 fois plus bas que ce que propose le marché actuellement. Le signal électrique donné par l'enzyme est environ 30 fois supérieur à tout support actuel pour biocapteurs, et peut être augmenté de manière exponentielle en augmentant son diamètre.

Le présent support pour biocapteurs est conçu pour une seule utilisation. Une fois épuisée la fonction de l'enzyme, celui-ci est rejeté.

Pour mieux comprendre l'objet de la présente invention, nous représentons sur les plans une forme préférentielle de réalisation pratique, susceptible de changements accessoires ne dénaturant pas leur fondement.

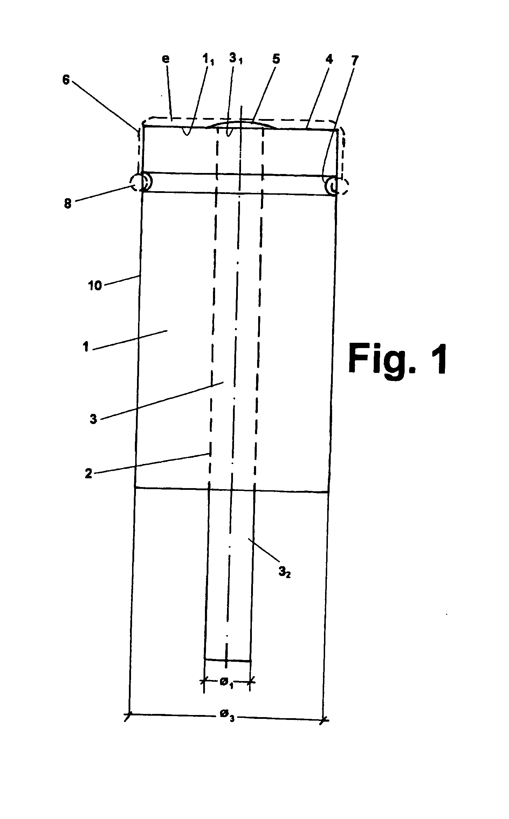

La figure 1 est une vue schématique en hauteur du biocapteur objet de l'invention.

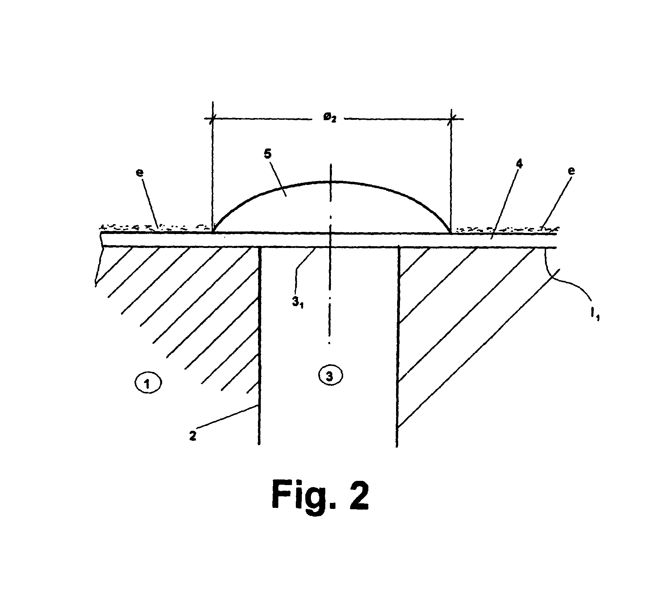

La figure 2 est une coupe très étendue de la zone de jonction tige (3) / couche en métal noble (4).

Nous décrivons ci-dessous un exemple de réalisation pratique, non limitatif, de la présente invention.

Le support du biocapteur comprend un cylindre (1) en plastique, de préférence en polychlorure de vinyle, avec un orifice passant (2) au centre, en faisant de celui-ci un tube. Le diamètre du cylindre (1) peut être de tout diamètre (Ø3) mais est de préférence de 10 mm. Dans cet orifice passant (2) est introduite une tige (3), par exemple, en acier inoxydable métallique, en cuivre, laiton, etc. (en laiton préférablement) avec l'unique objectif de transporter le flux d'électrons produit par les enzymes (e) qui seront placées sur la base supérieure (1₁) du cylindre en plastique (1) jusqu'au circuit électronique potentiostatique (non représenté).

Cette tige en métal (3) doit avoir un diamètre (Ø1) beaucoup plus petit que le diamètre du cylindre en plastique, 3 mm ou moins, de préférence Ø₁ < < Ø₃.

L'extrémité supérieure (3₁) de cette tige en laiton (3) est placée au ras du cylindre en plastique (1) et par l'autre extrémité (3₂) environ 5 mm ou tout autre mesure dépasse pour son amarrage postérieur au circuit électronique potentiostatique.

La zone du cylindre en plastique (1) au ras duquel la tige en laiton (3) a été placée est authentifiée/polie. Pour cela, on utilise de préférence une machine outil comme un treuil de révolution à basse vitesse pour que le cylindre en plastique ne fonde pas avec la friction. La surface ainsi polie doit être le plus lisse possible et ne pas brûler la matière plastique dans l'usinage à cause d'un excès de vitesse de la machine outil de tournage ou de polissage.

D'autre part, dans le cylindre en plastique (1) dans sa surface latérale (10) et à une distance de 2 mm de préférence à partir du bord supérieur de la zone polie, on réalise une fente (7) de forme toroïdale comme fixation future d'un joint (8) en gomme torique pour accrocher une membrane (6) perméable ou osmotique comme celles utilisées pour les dialyses.

Une fois le support du biocapteur tourné et préparé, on y dépose une couche en or de entre 1 et 1000 nanomètres, de préférence d'entre 30 et 100 nanomètres, en fonction du comportement d'autres enzymes quant à la réponse de génération du flux d'électrons ou de courant électrique face à la présence de la substance pour laquelle il a été conçu. Ce bain d'or est appliqué au moyen de sputtering avec un équipement commercial indiqué pour cela.

Avec ce recouvrement, toute la zone polie du cylindre en plastique (1) devient conductrice d'électricité, plus la tige en laiton (3).

Cet or, dont la couche est très mince, de l'ordre de quelques nanomètres, présente une impédance au passage de courant électrique qui peut être d'environ 10 ohms de la périphérie jusqu'au centre.

La zone trempée dans l'or problématique est celle du centre du cylindre en plastique (1), juste la zone où est logée la tige en laiton (3). Si l'enzyme (e) déposée sur l'or touche cette zone, un signal résiduel de base ou parasite non souhaité se produira, de l'ordre de 50 ou 100 fois le signal qui produirait l'enzyme (e) en présence de la substance pour laquelle il a été conçu, avec comme résultat un fonctionnement très anormal. Le signal électrique produit par l'enzyme serait masqué par ce signal parasite.

Pour éviter l'exposition de l'enzyme (e) à la zone centrale où se trouve la tige de laiton (3) sous le revêtement en or (4), on dépose un protecteur, une colle ou une petite goute (5) de résine époxydique de préférence, ou tout autre substance non conductrice adhérente au matériel du cylindre ou même un bouchon conçu pour cela. De cette manière, l'enzyme se déposera seulement dans toute la couronne polie du cylindre en plastique (1) trempée dans de l'or (4) excepté dans sa zone centrale couverte par une goute (5) de résine époxydique de préférence.

La goute ou le protecteur (5) sera de préférence d'un matériel adhérent à l'or.

L'enzyme (e) déposée sur ce support objet de brevet, est couverte par une membrane (6) perméable ou osmotique comme celles utilisées en dialyse, et on l'attache au côté du cylindre en plastique (1) au moyen d'un joint torique (8) en caoutchouc, qui est à son tour installé dans le renfoncement latéral (7) de forme torique qui a été pratiqué dans le cylindre en plastique (1) qui forme le support du biocapteur. La membrane perméable (6) doit être parfaitement serrée contre la face polie (1₁) du cylindre en plastique (1) pour immobiliser ainsi l'enzyme (e) et l'obliger à rester le plus près possible de la zone trempée dans de l'or de préférence.

Sans moyen de fixation/serrage de la membrane (6) comme par exemple ce renfoncement pour la mise en place du joint torique (8), la membrane perméable (6) pourrait se déplacer et faire que le biocapteur ainsi conçu ne fonctionne pas correctement, étant donné qu'il n'oblige pas l'enzyme (e) à rester le plus près possible de la zone trempée dans l'or.

On peut aussi remplacer la membrane perméable (6) par une membrane perméable liquide.

## Revendications

1. Supportez de biocapteur, comportant:
a) un cylindre (1) en matériel non-conducteur d'électricité, qui dispose d'un orifice (2) passant coaxial avec l'axe du cylindre (1)
b) une tige en métal (3) disposée dans cet orifice (2) avec son extrémité supérieure (3₁) placée au ras de la base supérieure (1₁) du cylindre (1)
c) une couche/revêtement en métal noble (4), d'entre 1 à 1000 nanomètres, disposée sur cette base supérieure (1₁)
d) un protecteur (5) d'un matériel non-conducteur d'électricité, adhérent à l'or et disposé sur la couche/revêtement en métal noble (4) dans la zone de jonction de cette couche (4) avec la tige en métal (3) et couvrant une surface de diamètre (Ø₂) supérieur au diamètre de la tige (3), de manière que l'enzyme se déposera dans toute la couche/revêtement en métal noble (4) excepté dans sa zone centrale couverte par le protecteur (5).

2. Support de biocapteur, selon la revendication 1, **caractérisé par le fait que** le cylindre (1) de matériel non-conducteur dispose sur sa surface latérale (10) de moyens de fixation d'une membrane (6).

3. Support de biocapteur, selon la revendication 1, **caractérisé par le fait que** la couche/revêtement (4) est en or, d'une épaisseur d'entre 30 et 100 nanomètres.

4. Support de biocapteur, selon la revendication 2, **caractérisé par le fait que** la membrane 6 peut être une membrane perméable ou une membrane perméable liquide.

5. Support de biocapteur, selon la revendication 1, **caractérisé par le fait que** le protecteur (5) est une goutte d'une résine adhérente à la couche/revêtement en métal noble (4).

## Claims

1. Biosensor support, which consists of:
a) a cylinder (1) of non-electroconductive material, having a through-hole (2) coaxial to the axis of the cylinder (1)
b) a metal rod (3) arranged in said opening (2) with its upper end (3₁) level with the top base (1₁) of the cylinder (1)
c) a layer/coating of noble metal (4), of between 1 and 1000 nanometres, arranged on said top base (1₁)
d) a protector (5) of a non-electroconductive material, adhering to the gold and arranged on the layer/coating of noble metal (4) in the area where said layer (4) joins the metal rod (3) and covering a surface with a diameter (Ø₂) larger than the diameter of the rod (3) so that the enzyme is deposited on all the layer of noble metal (4) except in its central area covered by the protector (5).

2. Biosensor support, according to claim 1, **characterized in that** the cylinder (1) of non-conductive material has means on its side surface (10) for fastening of a membrane (6).

3. Biosensor support, according to claim 1, **characterized in that** the layer/coating (4) is made of gold with a thickness of between 30 and 100 nanometres.

4. Biosensor support, according to claim 2, **characterized in that** the membrane 6 can be a permeable membrane or a liquid permeable membrane.

5. Biosensor support, according to claim 1, **characterized in that** the protector (5) is a drop of a resin that adheres to the layer/coating of noble metal (4).

## Patentansprüche

1. Biosensorträger, bestehend aus:
a) einem Zylinder aus nicht Elektrizität leitendem Material, der über eine durchgehende Öffnung (2) verfügt, koaxial zur Achse des Zylinders (1).
b) einem Metallstab (3) innerhalb dieser Öffnung (2) mit seinem oberen Ende (3₁) bodengleich zur oberen Basis (1₁) des Zylinders (1).
c) eine Schicht/ein Belag aus Edelmetall (4) von zwischen 1 bis 1000 Nanometern, angebracht auf dieser oberen Basis (1₁).
d) ein Protektor (5) eines nicht Elekrizität leitenden Materials, anhaftend an Gold und angebracht auf der Schicht/dem Belag aus Edelmetall (4) in dem Bereich der Verbindung dieser Schicht (4) mit dem Metallstab (3), und eine Oberfläche mit einem Durchmesser (Ø₂) bedeckend, der größer ist als der Durchmesser des Stabs (3), so dass das Enzym sich auf der gesamten Edelmetallschicht (4) festsetzt, mit Ausnahme seines mittleren Bereichs, der von dem Protektor (5) bedeckt ist.

2. Biosensorträger entsprechend Anspruch 1, **dadurch gekennzeichnet, dass** der Zylinder (1) aus nichtleitendem Material an seiner seitlichen Oberfläche (10) über Befestigungsmittel einer Membrane (6) verfügt.

3. Biosensorträger entsprechend Anspruch 1, **dadurch gekennzeichnet, dass** die Schicht/der Belag (4) aus Gold ist, mit einer Dicke zwischen 30 und 100 Nanometern.

4. Biosensorträger entsprechend Anspruch 2, **dadurch gekennzeichnet, dass** die Membrane (6) eine permeable Membrane sein kann oder eine flüssige permeable Membrane.

5. Biosensorträger entsprechend Anspruch 1, **dadurch gekennzeichnet, dass** der Protektor (5) ein Tropfen eines Harzes ist, der an der Schicht/dem Belag aus Edelmetall (4) haftet.
